(19) **Europäisches Patentamt / European Patent Office / Office européen des brevets**

(11) **EP 4 467 145 A1**

(12) **EUROPEAN PATENT APPLICATION**
published in accordance with Art. 153(4) EPC

(43) Date of publication:
**27.11.2024 Bulletin 2024/48**

(21) Application number: **23743525.0**

(22) Date of filing: **20.01.2023**

(51) International Patent Classification (IPC):
**A61K 31/7048** (2006.01)  **A61P 3/04** (2006.01)

(52) Cooperative Patent Classification (CPC):
**A61K 31/7048; A61P 3/04**

(86) International application number:
**PCT/KR2023/001008**

(87) International publication number:
**WO 2023/140682 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.01.2022 KR 20220008671**

(71) Applicant: **Daewoong Pharmaceutical Co., Ltd.**
**Hwaseong-si, Gyeonggi-do 18623 (KR)**

(72) Inventors:
• **HUH, Wan**
**Namyangju-si Gyeonggi-do 12111 (KR)**
• **LIM, Hyun-Woo**
**Yongin-si Gyeonggi-do 17066 (KR)**
• **CHOI, Ji Soo**
**Seoul 06310 (KR)**
• **PARK, Joon Seok**
**Yongin-si Gyeonggi-do 17000 (KR)**

(74) Representative: **Plasseraud IP**
**104 Rue de Richelieu**
**CS92104**
**75080 Paris Cedex 02 (FR)**

(54) **PHARMACEUTICAL COMPOSITION COMPRISING ENAVOGLIFLOZIN FOR PREVENTING OR TREATING OBESITY IN CANINE ANIMALS**

(57) The present invention relates to a pharmaceutical composition comprising enavogliflozin as an active ingredient for preventing or treating obesity in canine animals. The composition can be advantageously used for preventing or treating obesity in canine animals because it reduces the body condition score, body weight, fat thickness, body fat percentage, chest circumference, and waist circumference of obesity dogs, and has an excellent improvement effect on serological indicators.

[FIG. 4A]

EP 4 467 145 A1

**Description**

[Technical Field]

The present invention relates to a pharmaceutical composition for preventing or treating obesity in canine animals, which includes enavogliflozin.

[Background Art]

As the number of people raising dogs increases, food items such as meals and treats are becoming more diverse and abundant. However, this can lead to excessive eating and bad eating habits, inducing overweight and obesity in dogs.
Obesity in dogs causes comorbidities such as metabolic abnormalities, endocrine disorders, orthopedic disorders, cardiopulmonary diseases, genitourinary disorders, and tumors (J Nutr. 2006 Jul;136(7 Suppl):1940S-1946S.) and accelerates aging and shortens lifespans. For example, in Labrador retrievers, moderate obesity is known to shorten life expectancy by nearly two years (J Am Vet Med Assoc. 2002 May 1;220(9):1315-20.). Therefore, in order to live a long and healthy life with a companion dog, the dog's obesity must be actively managed.
Meanwhile, a sodium-glucose cotransporter 2 (SGLT2) inhibitor is an antihyperglycemic agent, which suppresses blood glucose elevation by reducing glucose reabsorption in the proximal nephron and increasing glucose release through an insulin-independent mechanism.

[Disclosure]

[Technical Problem]

The present inventors administered enavogliflozin to an obese dog to develop an obesity treatment for obese dogs, and confirmed an excellent effect of enavogliflozin in reducing a body condition score, a body weight, a fat thickness, a body fat percentage, a chest circumference and a waist circumference of an obese dog, and thus completed the present invention.
Therefore, the present invention is directed to providing a pharmaceutical composition for preventing or treating obesity in canine animals, which includes enavogliflozin as an active ingredient.

[Technical Solution]

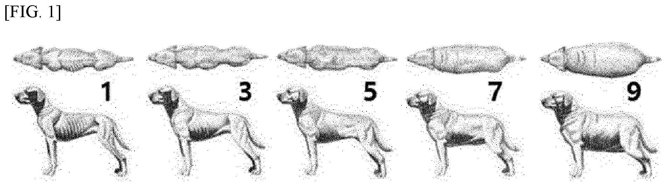

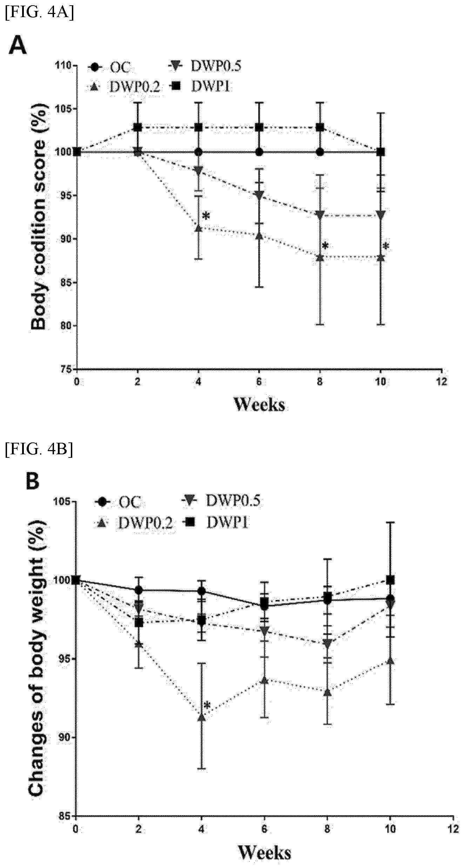

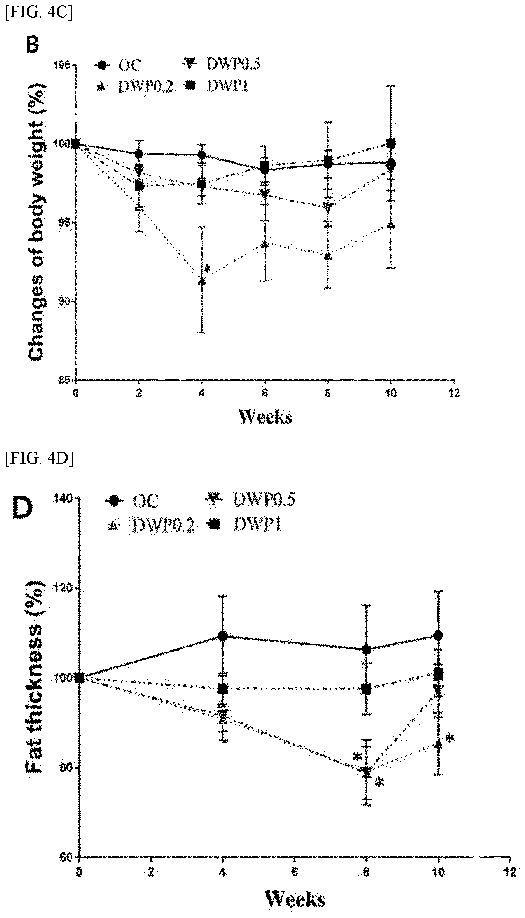

As in humans, obesity in canine animals such as companion dogs causes comorbidities such as metabolic abnormalities, endocrine disorders, osteoarthritis, patellar dislocation, intervertebral disk disease, cardiopulmonary diseases, and tumors.
The obesity degree of a companion dog may be determined based on a body condition score (BCS), classified into 5 or 9 levels. On the 5-level scale, level 3 indicates a standard body type, and on the 9-level scale, level 4 or 5 indicates a standard body type. On the 9-level scale, level 6 or 7 indicates mild obesity, level 8 indicates moderate obesity, and level 9 indicates severe obesity. Detailed evaluation criteria are presented in Table 1 and FIG. 1 of Examples below.
As confirmed in the following Examples, based on the BCS, enavogliflozin showed an effect in reducing an indicator such as BCS in an obese dog.
Accordingly, the present invention is directed to providing a pharmaceutical composition for preventing or treating obesity in canine animals, which includes enavogliflozin as an active ingredient.
A representative example of canine animals is a dog. In addition to a dog, the canine animals include a fox, a wolf, a coyote, a jackal, a wild dog, and a raccoon.
The term "prevention" used herein refers to all actions of inhibiting or delaying the occurrence of obesity by administration of the pharmaceutical composition of the present invention.
In addition, the term "treatment" used herein refers to all actions involved in alleviating or beneficially changing obesity by administration of the pharmaceutical composition of the present invention. More specifically, it may be judged whether an indicator selected from the group consisting of BCS, body weight, fat thickness, body fat percentage, chest circumference, waist circumference, and resting energy requirement is improved.
In one embodiment of the present invention, the canine animals may have a BCS of 6 or 7 or more. As described above, on the 9-level scale, when the BCS is 6 or more, it indicates mild obesity.
The content of enavogliflozin that can be used to prevent or treat obesity in canine animals is not particularly limited, and may be appropriately adjusted by the severity of obesity, body weight, age, sex, and the presence of other concomitant diseases.
In the following examples, the present inventors orally administered enavogliflozin to an obese dog at a dose of 0.2, 0.5, or 1 mg/kg for 8 weeks. As a result, it was confirmed that an indicator selected from the group consisting of BCS, body weight, fat thickness, body fat percentage, chest circumference, waist circumference, and resting energy requirement decreased (FIG. 4, and Tables 2 and 3).

Particularly, when enavogliflozin was administered to a normal dog at high, medium, and low doses for 13 or 39 weeks, the body weight decreased in a dose-dependent manner (FIGS. 2 and 3), whereas when enavogliflozin was administered to an obese dog at a dose of 0.2 mg/kg, an anti-obesity effect was the highest (FIG. 4, and Tables 2 to 5).

Accordingly, in one embodiment, enavogliflozin may be administered at 0.01 to 1 mg/kg once a day, preferably, at 0.05 to 0.7 mg/kg once a day, and more specifically, at 0.1 to 0.4 mg//kg once a day, but the present invention is not limited thereto.

When enavogliflozin is administered at 0.01 to 1 mg/kg once a day, it may be administered at 0.2, 0.5, or 1.0 mg/kg once a day, or when enavogliflozin is administered at 0.05 to 0.7 mg/kg once a day, it may be administered at 0.2 or 0.5 mg/kg once a day. In addition, when enavogliflozin is administered at a dose of 0.1 to 0.4 mg/kg once a day, it may be administered at 0.2 mg/kg once a day.

The administration period of enavogliflozin can be appropriately adjusted by a veterinarian based on the effect of preventing or treating obesity in canine animals according to the administration of enavogliflozin. The pharmaceutical composition, which includes enavogliflozin as an active ingredient, may be administered for at least 8 weeks to prevent or treat obesity in canine animals, but the present invention is not limited thereto.

Meanwhile, the administration of the pharmaceutical composition exhibits an effect of reducing an indicator selected from the group consisting of fasting blood sugar, serum insulin concentration, gamma-glutamyl transpeptidase (GGT), alanine aminotransferase (ALT), alkaline phosphatase (ALP), creatine phosphokinase (CPK), blood urea nitrogen (BUN), creatinine (CRE), and C-reactive protein (CRP) in obese dogs. This means that enavogliflozin not only has an anti-obesity effect, but can also improve hyperglycemia, hyperlipidemia, and fatty liver disease, providing a great advantage in obesity treatment.

Accordingly, canine animals that requires administration of the pharmaceutical composition may additionally suffer from a metabolic disease selected from the group consisting of hyperglycemia, hyperlipidemia, and fatty liver, in addition to obesity, but the present invention is not limited thereto.

Meanwhile, the pharmaceutical composition containing enavogliflozin according to the present invention may be administered orally or parenterally depending on the desired method, but the present invention is not limited thereto. A formulation for oral administration may have various forms, such as a syrup, a tablet, a capsule, a cream, and a lozenge. A syrup formulation will generally contain a suspension or solution of a compound in a liquid carrier, which optionally contains a flavoring or coloring agent, or a salt thereof, for example, ethanol, peanut oil, olive oil, glycerin, or water.

When the composition is prepared in a tablet form, any one of the pharmaceutical carriers generally used for preparing a solid preparation may be used. Examples of such carriers are magnesium stearate, tera alba, talc, gelatin, acacia, stearic acid, starch, lactose, and sucrose. When the composition is prepared in a capsule form, one of general encapsulation procedures may be used, and for example, the above-described carrier may be used in a soft gelatin capsule shell. The composition may be prepared in the form of a soft gelatin shell capsule. Any one of the pharmaceutical compositions generally used in a dispersion or suspension may be prepared using aqueous gum, cellulose, silicate, or oil. Formulations for intramuscular or subcutaneous administration may be in liquid forms, for example, solutions, suspensions and emulsions, which include aqueous solvents such as water, saline, and Ringer's solution, or lipophilic solvents such as fatty oils, seasame oil, corn oil, and synthetic fatty acid esters.

The present invention is also directed to providing a method of treating obesity in canine animals, which includes administering a pharmaceutical composition including enavogliflozin as an active ingredient to a canine animal in need of treatment.

Among the terms or elements mentioned in the method for treating obesity, those mentioned in the description of the composition for preventing or treating obesity in canine animals are used in the same meaning as in the description of the composition for preventing or treating obesity in canine animals.

[Advantageous Effects]

A pharmaceutical composition for preventing or treating obesity in canine animals according to the present invention, which includes enavogliflozin as an active ingredient, can reduce a body condition score, a body weight, a fat thickness, a body fat percentage, a chest circumference and a waist circumference in obese dogs, and also have excellent effect of improving serological indicators, and thus can be useful for the prevention or treatment of obesity in canine animals.

[Description of Drawings]

FIG. 1 shows the body types of a companion dog according to body condition scores.

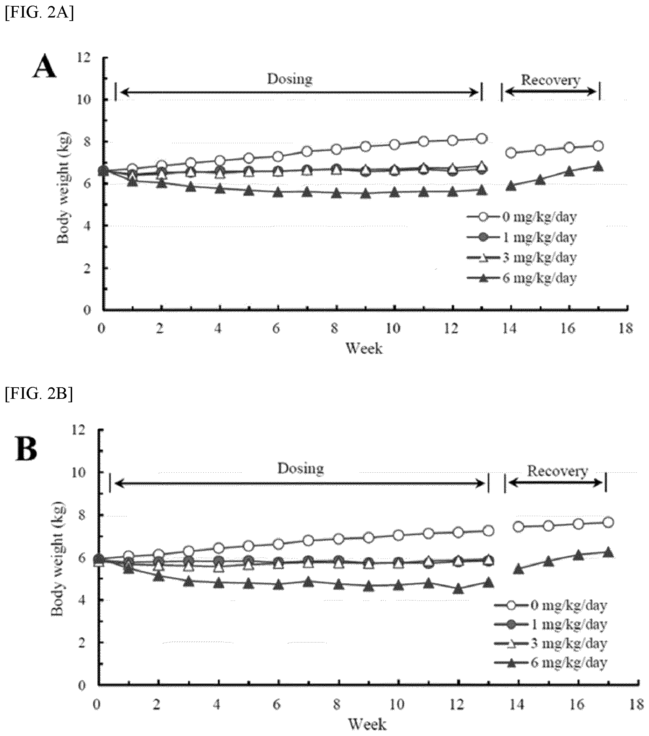

FIG. 2A shows the result of confirming changes in body weight after administering enavogliflozin to normal male dogs at different doses for 13 weeks.

FIG. 2B shows the result of confirming changes in body weight after administering enavogliflozin to normal female dogs at different doses for 13 weeks.

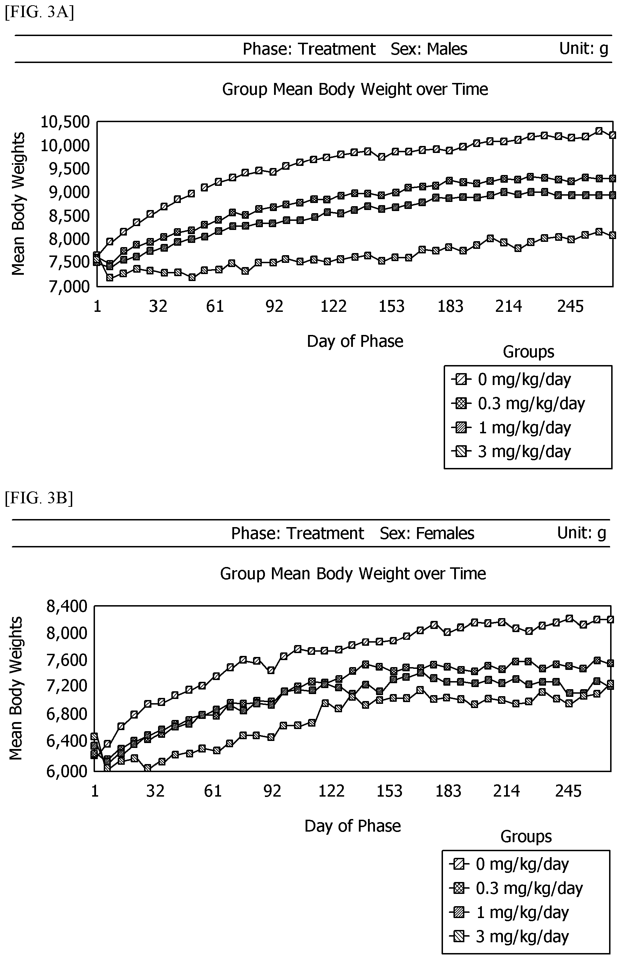

FIG. 3A shows the result of confirming changes in body weight after administering enavogliflozin to normal male dogs at different doses for 39 weeks.

FIG. 3B shows the result of confirming changes in body weight after administering enavogliflozin to normal female dogs at different doses for 39 weeks.

FIG. 4A shows the result of confirming changes in body condition score after administering enavogliflozin to obese dogs at different doses.

FIG. 4B shows the result of confirming changes in body weight after administering enavogliflozin to obese dogs at different doses.

FIG. 4C shows the result of confirming changes in body fat percentage after administering enavogliflozin to obese dogs at different doses.

FIG. 4D shows the result of confirming changes in fat thickness after administering enavogliflozin to obese dogs at different doses.

[Modes of the Invention]

Hereinafter, one or more embodiments will be described in further detail with reference to examples. However, these examples are provided to exemplify one or more embodiments, and the scope of the present invention is not limited to the following examples.

**Experimental methods**

**1. Non-clinical toxicity test**

Changes in body weight were confirmed after administering enavogliflozin to four female and four male 6-month-old normal beagles at 0 (control, gelatin capsule), 1, 3, and 6 mg/kg/day for 13 weeks. The above amounts of enavogliflozin were charged in a gelatin capsule and administered daily.

In addition, changes in body weight were confirmed after administering enavogliflozin to four female and four male 6-month-old normal beagles at 0 (control, gelatin capsule), 0.3, 1, and 3 mg/kg/day for 39 weeks.

**2. Screening, grouping, and monitoring of clinical trial animals**

Adult dogs which were naturally obese but otherwise healthy were selected regardless of breed, sex, and altered status, and informed consent was obtained from the dog owners for participation in the clinical trial.

As a result of physical examination of all dogs (including body weight and BCS (a total of 9 levels), FIG. 1 and Table 1), those diagnosed as obese were selected. The BCS used to select dogs is classified into a total of 9 levels, and dogs with a BCS of level 6 or higher were selected, and detailed criteria are presented in Table 1 and FIG. 1.

[Table 1]

| Characteristics | Scores | Evaluation criteria |
|---|---|---|
| Emaciation | 1 | The prominences of the ribs, lumbar spine, pelvic bones, and all bones are easily visible even from a distance, body fat is not visible, and muscle loss is clearly visible. |
| | 2 | Ribs, lumbar, and pelvic bones are easily visible; fat is not palpable; some bone prominences are visible; and a small reduction in muscle mass is evident. |
| | 3 | The ribs are easily palpated and might be visible with little palpable fat. The tip of the lumbar spine is visible, pelvic bones are prominent, and the waist and abdomen are obviously tucked. |

(continued)

| Characteristics | Scores | Evaluation criteria |
|---|---|---|
| Normal | 4 | The ribs are covered with minimal fat and are easily palpated, the waist is easily identified when viewed from the tope, and the abdominal tuck is evident when viewed from the side. |
| | 5 | The ribs are palpable without excess fat, the waist is visible behind the ribs when viewed from above, and the abdomen is tucked when viewed from the side. |
| Obesity | 6 | The ribs are palpable with a slight excess of fat, the waist is distinguishable but not prominent, and an abdominal tuck is evident. |
| | 7 | Ribs are covered in fat and difficult to palpate. Fat accumulation is seen in the lumbar spine and the base of the tail. It is difficult to distinguish the waist. Abdominal tuck can be present. |
| | 8 | Heavy covering of fat over the ribs, which cannot be palpated or are palpable only when pressure is applied; extensive fat accumulation in the lumbar spine and base of the tail, causing the flesh to fold. No distinction between the waist and abdomen. Obvious abdominal swelling. |
| | 9 | A very large amount of fat accumulates in the neck, spine, and tail of the body, causing the flesh to fold, and the waist and stomach are indistinguishable. Fat accumulates in the limbs, and there is abdominal swelling. |

The selected dogs were generally healthy with no underlying diseases such as abnormalities in the liver, heart, and/or kidneys, and only one dog per household was analyzed to ensure individual feeding.

Dogs used for breeding or receiving long-acting steroids, drugs that affect endocrine conditions (e.g., lipid improving agents, cholesterol inhibitors, and diabetes drugs), or drugs that affect body weight or energy consumption (e.g., phenobarbital) within 30 days prior to the start of the clinical trial were excluded.

A total of 20 dogs were selected and divided into 4 groups with 5 dogs in each group: a normal obese control (OC); hereinafter, referred to as a control (OC)), an enavogliflozin 0.2-administered group, an enavogliflozin 0.5-administered group, and an enavogliflozin 1-administered group. The control was fed regular feed, and the enavogliflozin-administered groups were fed regular feed supplemented with enavogliflozin at doses of 0.2, 0.5, or 1 mg/kg, respectively. The feed was supplemented with enavogliflozin once a day for 8 weeks and given orally.

Dog breeds participating in the clinical trials are as follows: Shetland Sheepdog (5/20), Maltese (3/20), Pompitz (2/20), Bichon Frise (1/20), Border Collie (1/20), Cocker Spaniel (1/ 20), Japanese Spitz (1/20), Labrador Retriever (1/20), Poodle (1/20), Shih Tzu (1/20), Wire Fox Terrier (1/20), Yorkshire Terrier (1/20), and Mixed (1/20).

Among the 20 dogs, 10 were male and all of them were neutered, and the other 10 were female and 7 of them were neutered. The age range was 3 to 10 years, and an average age was 7.15. Among the 20 dogs, 9 were 6 years old or younger, 8 were 7 to 9 years old, and 3 were 10 years old or older.

All dogs participating in the clinical trials were examined by a veterinarian once a week until the end of the trial.

### 3. Measurement of body weight, chest circumference, waist circumference, and resting energy requirement

Body weights (BW), chest circumferences, and waist circumferences were measured immediately before the first intake of enavogliflozin-supplemented feed, and once every two weeks for the next 10 weeks. The chest circumferences and waist circumferences were measured four times each at the thickest part of the chest and the thinnest part between the chest and hind legs, and an average size was calculated. Resting energy requirement (RER) was calculated using Equation 1 in the reference document (Brooks et al., Journal of the American Animal Hospital Association 50, 1-11. (2014)).

[Equation 1]

$$RER \text{ (kcal/day)} = 70 \times (\text{Initial body weight})^{0.75}$$

### 4. Measurement of fat thickness and body fat

Fat thicknesses were evaluated by measuring the perpendicular distance from the T10 spinal process to the skin by radiography (Titan 2000 X-ray system, COMED Medical Systems Co., Ltd., Seoul, Korea) on the first day of enavogliflozin administration, and on weeks 4, 8, and 10 of administration. Body fat was measured with a body fat meter every two weeks for a total of 10 weeks after the start of enavogliflozin administration.

**5. Blood, serum, and urine tests**

Hematological tests were performed on the day before the first administration of enavogliflozin and once every two weeks for the next 10 weeks. A portion of the blood collected on the day of the test was stored in a CBC tube containing the anticoagulant EDTA-2K to assess a red blood cell (RBC) count, hematocrit (Hct), a hemoglobin (Hb) concentration, a white blood cell (WBC) count, and a platelet (PLT) count using an automated hematology analyzer (ADVIA 2120i™, Siemens Healthcare Diagnostics, Vienna, Austria).

Biological analysis was performed as follows. A portion of the blood collected on the day of the test was transferred to a vacuum tube containing a coagulation activator, left at room temperature for 15 to 20 minutes to solidify, and then centrifuged at 3000 rpm, thereby obtaining serum. Serum biochemical parameters were evaluated using a Hitachi 7180 device (Hitachi, Tokyo, Japan). The evaluated biochemical parameters are aspartate aminotransferase (AST), alanine aminotransferase (ALT), alkaline phosphatase (ALP), creatine phosphokinase (CPK), total bilirubin (TBIL), glucose (GLU), total cholesterol (TC), triglycerides (TG), total protein (TP), albumin (ALB), gamma-glutamyl transpeptidase (GGT), blood urea nitrogen (BUN), creatinine (CRE), inorganic phosphorus (IP), calcium (Ca), and C-reactive protein (CRP).

Serum insulin concentrations were measured 2, 4, 6, 8, and 10 weeks after the start of enavogliflozin administration using a Quantikine Canine Insulin ELISA kit (Catalog no. DINS00, R&D Systems, Minneapolis, MN, USA) according to the manufacturer's protocol. Urine biochemical parameters were evaluated using urine test strips 2, 4, 6, and 10 weeks after the start of enavogliflozin administration, and urinary glucose (GLU) excretion was measured using UriSCAN® Strip (YD Diagnostics Corp., Yong-in, Korea).

**6. Statistical analysis**

Prism 5.03 software (GraphPad Software Inc., San Diego, CA, USA) was used for the statistical analysis of data. Test results were expressed as mean±SEM. The statistical method used a paired Student's t-test or Wilcoxon matched pairs test, and the significance level was set at $p < 0.05$.

**Experimental results**

**1. Non-clinical toxicity test**

As a result of repeated oral administration of enavogliflozin for 13 weeks, body weight decreased compared to the control. Specifically, at doses of 1, 3, and 6 mg/kg/day, the body weights of the male beagles decreased by 17.7%, 16%, and 29.9% (FIG. 2A), respectively, and the body weights of the female beagles decreased by 19.6%, 18.5%, and 33.2%, respectively, indicating that body weight decreased in a dose-dependent manner when enavogliflozin was administered (FIG. 2B).

Even when enavogliflozin was repeatedly administered orally for 39 weeks, at doses of 1, 3, and 6 mg/kg/day, the body weights of the male beagles decreased 9.1%, 12.7%, and 21.0%, respectively (FIG. 3A), and the body weights of the female beagles decreased 7.6%, 11.8%, and 11.3%, respectively (FIG. 3B).

**2. Changes in BCS, body weight, body fat, fat thickness, chest circumference, and waist circumference**

The effects of enavogliflozin administration on BCS, body weight, body fat content, fat thickness, chest circumference, and waist circumference in obese dogs were evaluated.

The BCS in the enavogliflozin 0.2-administered group (DWP0.2) was significantly lower than the control (OC) at 4, 8, and 10 weeks after the start of administration ($p < 0.05$), and the BCS in the enavogliflozin 0.5-administered group (DWP0.5) also decreased during the entire experimental period. The BCS of the enavogliflozin 1-administered group (DWP1.0) slightly increased compared to the control (OC), but was lower than the control (OC) at the end of the experiment (FIG. 4A).

The boy weight in the enavogliflozin 0.2-administered group was significantly lower than the control (OC) from 4 weeks after the start of administration ($p < 0.05$), and the weight change was also the lowest among all groups. Similar weight loss also occurred in the enavogliflozin 0.5-administered group with the body weight decreasing at week 2 and week 4 of the trial and then slowly increasing (FIG. 4B). There were no significant differences in body fat percentage in all groups, but it was the lowest in the enavogliflozin 0.2-administered group (FIG. 4C). The fat thickness decreased in all administration groups, and such a change was significant at week 8 and week 10 in the enavogliflozin 0.2-administered group (FIG. 4D). Both the chest circumference and the waist circumference decreased in all enavogliflozin-administered groups (Table 2). In the following Table 2, changes in chest circumference and waist circumference are shown.

[Table 2]

| Weeks | Change rates of chest circumference (%) | | | |
|---|---|---|---|---|
| | Control (OC) | Enavogliflozin 0.2-administered group | Enavogliflozin 0.5-administered group | Enavogliflozin 1-administered group |
| 0 | 100.00±0.00 | 100.00±0.00 | 100.00±0.00 | 100.00±0.00 |
| 2 | 99.39±3.13 | 99.20±3.44 | 98.19±4.31 | 94.79±6.67 |
| 4 | 99.64±3.44 | 97.16±2.74 | 97.16±3.23 | 99.81±2.43 |
| 6 | 96.92±5.74 | 95.08±3.96 | 95.60±4.55 | 100.54±4.98 |
| 8 | 96.99±4.44 | 96.08±2.60 | 93.90±3.11 | 99.20±4.49 |
| 10 | 99.52±6.24 | 99.18±3.01 | 96.43±2.76 | 100.24±5.15 |
| | Change rates of waist circumference (%) | | | |
| 0 | 100.00±0.00 | 100.00±0.00 | 100.00±0.00 | 100.00±0.00 |
| 2 | 101.93±8.89 | 100.53±4.71 | 99.57±1.71 | 95.97±11.67 |
| 4 | 104.05±6.64 | 97.39±12.15 | 98.45±4.94 | 99.18±8.09 |
| 6 | 96.08±7.08 | 98.96±15.47 | 96.30±3.83 | 103.75±10.54 |
| 8 | 102.61±6.40 | 98.89±11.98 | 93.84±5.54 | 99.76±12.53 |
| 10 | 103.54±2.93 | 101.98±10.78 | 96.00±4.72 | 101.33±14.44 |

## 3. Changes in feed intake and resting energy requirement

The feed intake at week 10 of enavogliflozin administration was 101.35±2.56 in the control (OC), 166.59±4.72 in the enavogliflozin 0.2-administered group, 98.47±1.44 in the enavogliflozin 0.5-administered group, and 123.15±2.45% in the enavogliflozin 1.0-administered group, compared to the initial feed intake. The feed intake during the 10-week experimental period significantly increased in the enavogliflozin 0.2-administered group and the enavogliflozin 1-administered group ($p < 0.001$), and decreased in the enavogliflozin 0.5-administered group, compared to the control (OC) (Table 3).

As the result of analyzing the resting energy requirement (RER) at 4 weeks and 8 weeks after the start of enavogliflozin administration, compared to the control (OC), RER was significantly lower in the enavogliflozin 0.2-administered group ($p < 0.05$; Table 3).

The changes in feed intake and RER of each experimental group during the clinical trial period are shown in Table 3. The results are expressed as mean ± standard deviation, and * indicates a significant result at $p < 0.05$ compared to the control (OC).

[Table 3]

| Weeks | Change rates of food consumption (%) | | | |
|---|---|---|---|---|
| | Control (OC) | Enavogliflozin 0.2-administered group | Enavogliflozin 0.5-administered group | Enavogliflozin 1-administered group |
| 0 | 100.00±0.00 | 100.00±0.00 | 100.00±0.00 | 100.00±0.00 |
| 2 | 102.10±1.31 | 126.52±5.48*** | 95.13±1.67*** | 100.80±4.20 |
| 4 | 101.38±1.25 | 146.64±5.37*** | 92.34±2.10*** | 116.02±3.09*** |
| 6 | 99.75±1.19 | 153.71±4.11*** | 92.30±1.60*** | 121.84±2.50*** |
| 8 | 98.74±1.41 | 168.32±4.80*** | 93.64±1.67* | 121.88±2.36*** |
| 10 | 101.35±2.56 | 166.59±4.72*** | 98.47±1.44 | 123.15±2.45*** |

(continued)

| | Change rates of resting energy requirement (%) | | | |
|---|---|---|---|---|
| 0 | 100.00±0.00 | 100.00±0.00 | 100.00±0.00 | 100.00±0.00 |
| 2 | 99.51±1.39 | 96.98±2.69 | 98.63±0.72 | 97.75±2.32 |
| 4 | 99.48±1.10 | 93.41±5.79 | 97.93±1.00 | 98.11±2.18 |
| 6 | 98.74±1.32 | 95.21±4.10 | 97.54±2.73 | 98.95±2.07 |
| 8 | 99.04±1.45 | 94.64±3.59* | 96.92±2.00 | 99.19±4.05 |
| 10 | 99.12±1.77 | 96.15±4.75 | 98.79±2.30 | 99.97±6.15 |

### 4. Blood and serum biochemical parameters

During the 10-week trial period, there were no significant differences in hematological indicators between the administration groups, except for the platelet (PLT) count. The platelet (PLT) count of the enavogliflozin 1-administered group was significantly lower than that of the control (OC) (p<0.05), and the serum total cholesterol (TC) and triglyceride (TG) concentration of the enavogliflozin 0.2-administered group were lower than those of the control (OC) (Table 4).

In all administration groups, fasting glucose and serum insulin concentrations decreased compared to the control (OC), but the glucose (GLU) concentration was significantly lower only in the enavogliflozin 1-administered group, and the insulin concentration was significantly lower only in the enavogliflozin 0.5-administered group, compared to the control (OC) (Table 4).

The serum triglyceride (TG) concentrations of the enavogliflozin-administered groups were 142.00±95.55 mg/dL for the enavogliflozin 0.2-administered group, 133.86±70.19 mg/dL for the enavogliflozin 0.5-administered group, and 282.60±419.03 mg/dL for the enavogliflozin 1-administered group, which were significantly lower than that of the control (361.60±622.34 mg/dL). In addition, the concentrations of serum gamma-glutamyl transpeptidase (GGT), alanine aminotransferase (ALT), alkaline phosphatase (ALP), creatine phosphokinase (CPK), and C-reactive protein (CRP) significantly decreased in the enavogliflozin 0.2-administered group, compared to the control (OC) (Table 4).

In Table 4, the results are expressed as mean ± standard deviation, and * indicates a significant result at p<0.05 by a post hoc test according to one-way ANOVA compared to the control (OC).

[Table 4]

| | Control (OC) | Enavogliflozin 0.2-administered group | Enavogliflozin 0.5-administered group | Enavogliflozin 1-administered group |
|---|---|---|---|---|
| WBC (X10$^3$ μL) | 7.93±2.83 | 11.12±3.69 | 7.75±2.04 | 10.01±3.35 |
| RBC (×10$^6$ μL) | 6.90±0.99 | 6.92±0.79 | 72.22±0.32 | 6.55±1.08 |
| Hb (g/dL) | 16.36±1.98 | 16.34±2.68 | 16.40±0.70 | 16.16±2.15 |
| Hct (%) | 47.94±7.61 | 49.86±7.08 | 50.26±1.94 | 46.76±6.13 |
| Platelet (×10$^3$/μL) | 422.60±12.98 | 392.20±11.16 | 307.40±6.02 | 299.10±7.65* |
| Glu (mg/dL) | 113.80±10.85 | 107.20±10.85 | 97.77±10.19 | 94.00±13.47 |
| Insulin (ng/mL) | 133.27±16.43 | 126.60±15.45 | 99.82±18.48* | 110.75±21.96 |
| TC (mg/dL) | 236.40±69.21 | 183.84±36.01 | 298.00±71.14 | 309.00±129.40 |
| TG (mg/dL) | 361.60±622.34 | 142.00±95.55 | 133.86±70.19 | 282.60±419.03 |
| TP (mg/dL) | 6.48±0.66 | 6.78±0.66 | 6.43±0.32 | 6.14±0.54 |
| ALB (g/dL) | 3.04±0.30 | 3.48±0.42 | 2.95±0.19 | 2.92±0.29 |
| GGT (IU/L) | 13.20±14.55 | 7.80±1.64 | 9.46±5.37 | 21.60±29.36 |
| ALT(IU/L) | 50.60±29.33 | 18.84±4.00* | 36.80±14.43* | 38.60±19.72 |
| AST(IU/L) | 44.80±16.21 | 31.00±1.83 | 31.84±12.92 | 110.00±167.92 |
| ALP(IU/L) | 211.60±199.74 | 84.60±63.96* | 146.20±121.67 | 186.20±184.59 |

(continued)

|  | Control (OC) | Enavogliflozin 0.2-administered group | Enavogliflozin 0.5-administered group | Enavogliflozin 1-administered group |
|---|---|---|---|---|
| CPK(IU/L) | 129.40±35.82 | 111.12±30.97* | 136.50±42.99 | 260.40±126.79 |
| BUN (mg/dL) | 15.12±6.30 | 18.70±10.29 | 22.25±8.64 | 23.44±13.17 |
| CRE (mg/dL) | 1.20±0.31 | 0.78±0.21* | 0.82±0.15 | 0.92±0.36 |

Urinary glucose (GLU) excretion was higher in the enavogliflozin-administered groups, particularly, the enavogliflozin 0.2-administered group (Table 5).

[Table 5]

| Urine test - Glucose detection (2 weeks) | | | | | |
|---|---|---|---|---|---|
| | | Control (OC) | Enavogliflozin 0.2-administered group | Enavogliflozin 0.5-administered group | Enavogliflozin 1-administered group |
| 2 weeks | -, 0 | 5 | 0 | 0 | 0 |
| | +, 1 | 0 | 3 | 1 | 0 |
| | ++, 2 | 0 | 0 | 1 | 0 |
| | +++, 3 | 0 | 1 | 0 | 2 |
| | +++, 4 | 0 | 1 | 3 | 3 |
| Urine test - Glucose detection (4 weeks) | | | | | |
| 4 weeks | -, 0 | 5 | 0 | 0 | 0 |
| | +, 1 | 0 | 2 | 0 | 0 |
| | ++, 2 | 0 | 1 | 0 | 0 |
| | +++, 3 | 0 | 1 | 0 | 0 |
| | +++, 4 | 0 | 1 | 5 | 5 |
| Urine test - Glucose detection (6 weeks) | | | | | |
| 6 weeks | -, 0 | 5 | 0 | 0 | 0 |
| | +, 1 | 0 | 3 | 0 | 0 |
| | ++, 2 | 0 | 0 | 0 | 0 |
| | +++, 3 | 0 | 1 | 0 | 0 |
| | +++, 4 | 0 | 1 | 5 | 5 |
| Urine test - Glucose detection (8 weeks) | | | | | |
| 8 weeks | -, 0 | 5 | 0 | 0 | 0 |
| | +, 1 | 0 | 1 | 0 | 0 |
| | ++, 2 | 0 | 2 | 1 | 0 |
| | +++, 3 | 0 | 1 | 0 | 1 |
| | +++, 4 | 0 | 1 | 4 | 5 |

(continued)

| Urine test - Glucose detection (10 weeks) | | | | | |
|---|---|---|---|---|---|
| 10 weeks | -, 0 | 5 | 0 | 1 | 3 |
| | +, 1 | 0 | 1 | 1 | 0 |
| | ++, 2 | 0 | 1 | 1 | 0 |
| | +++, 3 | 0 | 2 | 1 | 1 |
| | +++, 4 | 0 | 1 | 1 | 1 |

## 5. Conclusion

8-week enavogliflozin administration reduced BCS, body weight, body fat percentage, fat thickness, and chest and waist circumferences in obese dogs, and when the feed was supplemented with 0.2 mg/kg of enavogliflozin, the most effective results were shown.

In addition, in the enavogliflozin-administered groups, blood sugar decreased, urinary glucose excretion increased, and all of the concentrations of serum total cholesterol (TC), triglyceride (TG), aspartate aminotransferase (AST), alanine aminotransferase (ALT), creatine phophokinase (CPK), blood urea nitrogen (BUN), and creatine (CRE), which were increased in obese individuals, were decreased. Increased concentrations of total cholesterol (TC) and triglyceride (TG) are indicators of hyperlipidemia, and increased concentrations of blood urea nitrogen (BUN) and creatine (CRE) can be seen in kidney failure. Increased concentrations of aspartate aminotransferase (AST), alanine aminotransferase (ALT), and creatine phosphokinase (CPK) are indicators of fatty liver.

Therefore, it can be seen that enavogliflozin improves the condition of obese dogs, and also has the effect of improving hyperglycemia, hyperlipidemia, and fatty liver.

## Claims

1.  A pharmaceutical composition for use in preventing or treating obesity in canine animals, comprising: enavogliflozin as an active ingredient.

2.  The pharmaceutical composition for use according to claim 1, wherein the canine animals have a body condition score (BCS) of 6 or more.

3.  The pharmaceutical composition for use according to claim 1, wherein the canine animals have a body condition score (BCS) of 7 or more.

4.  The pharmaceutical composition for use according to claim 1, wherein the enavogliflozin is administered at 0.01 to 1 mg/kg once a day.

5.  The pharmaceutical composition for use according to claim 1, wherein the enavogliflozin is administered at 0.05 to 0.7 mg/kg once a day.

6.  The pharmaceutical composition for use according to claim 1, wherein the enavogliflozin is administered at 0.1 to 0.4 mg/kg once a day.

7.  The pharmaceutical composition for use according to claim 1, which is administered for at least 8 weeks.

8.  The pharmaceutical composition for use according to claim 1, which improves one or more indicators selected from the group consisting of body condition score, body weight, fat thickness, body fat percentage, chest circumference, waist circumference, and resting energy requirement.

9.  The pharmaceutical composition for use according to claim 1, which reduces one or more indicators selected from the group consisting of fasting blood sugar, serum insulin concentration, gamma-glutamyl transpeptidase (GGT), alanine aminotransferase (ALT), alkaline phosphatase (ALP), creatine phosphokinase (CPK), and C-reactive protein (CRP).

10. The pharmaceutical composition for use according to claim 1, which is administered orally or parenterally.

11. The pharmaceutical composition for use according to claim 1, wherein the canine animals additionally suffer from a metabolic disease selected from the group consisting of hyperglycemia, hyperlipidemia, and fatty liver.

12. A method of treating obesity in canine animals, comprising:
    administering the pharmaceutical composition of claim 1 to a canine animal in need of treatment.

[FIG. 1]

[FIG. 2A]

[FIG. 2B]

[FIG. 3A]

Phase: Treatment    Sex: Males                    Unit: g

## Group Mean Body Weight over Time

Groups
- ⊡ 0 mg/kg/day
- ⊠ 0.3 mg/kg/day
- ▨ 1 mg/kg/day
- ◫ 3 mg/kg/day

[FIG. 3B]

Phase: Treatment    Sex: Females                    Unit: g

## Group Mean Body Weight over Time

Groups
- ⊡ 0 mg/kg/day
- ⊠ 0.3 mg/kg/day
- ▨ 1 mg/kg/day
- ◫ 3 mg/kg/day

[FIG. 4A]

[FIG. 4B]

[FIG. 4C]

[FIG. 4D]

## INTERNATIONAL SEARCH REPORT

International application No.

**PCT/KR2023/001008**

### A. CLASSIFICATION OF SUBJECT MATTER

**A61K 31/7048**(2006.01)i; **A61P 3/04**(2006.01)i

According to International Patent Classification (IPC) or to both national classification and IPC

### B. FIELDS SEARCHED

Minimum documentation searched (classification system followed by classification symbols)

A61K 31/7048(2006.01); A61K 31/351(2006.01); A61K 31/7034(2006.01); A61K 38/28(2006.01); A61K 47/10(2006.01); A61K 9/00(2006.01); C07D 309/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이나보글리플로진(enavogliflozin), 개과(canidae), 비만(obesity), 대사 질환 (metabolic disease), SGLT2

### C. DOCUMENTS CONSIDERED TO BE RELEVANT

| Category* | Citation of document, with indication, where appropriate, of the relevant passages | Relevant to claim No. |
|---|---|---|
| Y | KR 10-2016-0108556 A (BOEHRINGER INGELHEIM VETMEDICA GMBH) 19 September 2016 (2016-09-19)<br>See paragraph [0046]; and claims 1-2, 9 and 15. | 1-12 |
| Y | KR 10-2014-0022086 A (GREEN CROSS CORPORATION) 21 February 2014 (2014-02-21)<br>See example 172; and table 1, compound E172. | 1-12 |
| A | KR 10-2018-0039727 A (BOEHRINGER INGELHEIM VETMEDICA GMBH) 18 April 2018 (2018-04-18)<br>See entire document. | 1-12 |
| A | KR 10-2017-0028452 A (BOEHRINGER INGELHEIM INTERNATIONAL GMBH) 13 March 2017 (2017-03-13)<br>See entire document. | 1-12 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 April 2023** | **26 April 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/001008**

### C. DOCUMENTS CONSIDERED TO BE RELEVANT

| Category* | Citation of document, with indication, where appropriate, of the relevant passages | Relevant to claim No. |
|---|---|---|
| A | IDRIS, I. et al. Sodium–glucose co-transporter-2 inhibitors: an emerging new class of oral antidiabetic drug. Diabetes, Obesity and Metabolism. 2009, vol. 11, pp. 79–88. See entire document. | 1-12 |

Form PCT/ISA/210 (second sheet) (July 2022)

# EP 4 467 145 A1

**INTERNATIONAL SEARCH REPORT**
**Information on patent family members**

International application No.

**PCT/KR2023/001008**

| Patent document cited in search report | | | Publication date (day/month/year) | Patent family member(s) | | | Publication date (day/month/year) |
|---|---|---|---|---|---|---|---|
| KR | 10-2016-0108556 | A | 19 September 2016 | AU | 2015-208291 | A1 | 23 June 2016 |
| | | | | AU | 2015-208291 | B2 | 05 December 2019 |
| | | | | AU | 2020-201168 | A1 | 05 March 2020 |
| | | | | AU | 2020-201168 | B2 | 25 March 2021 |
| | | | | CA | 2932674 | A1 | 30 July 2015 |
| | | | | CN | 105960242 | A | 21 September 2016 |
| | | | | CN | 115282284 | A | 04 November 2022 |
| | | | | DK | 3096765 | T3 | 04 March 2019 |
| | | | | EA | 032558 | B1 | 28 June 2019 |
| | | | | EA | 201600535 | A1 | 30 November 2016 |
| | | | | EA | 201891406 | A1 | 28 December 2018 |
| | | | | EP | 3096765 | A1 | 30 November 2016 |
| | | | | EP | 3096765 | B1 | 05 December 2018 |
| | | | | EP | 3485890 | A1 | 22 May 2019 |
| | | | | ES | 2712860 | T3 | 16 May 2019 |
| | | | | JP | 2017-503824 | A | 02 February 2017 |
| | | | | JP | 2018-021079 | A | 08 February 2018 |
| | | | | JP | 2020-029460 | A | 27 February 2020 |
| | | | | JP | 6239775 | B2 | 29 November 2017 |
| | | | | JP | 7022106 | B2 | 17 February 2022 |
| | | | | KR | 10-2022-0097538 | A | 07 July 2022 |
| | | | | KR | 10-2414283 | B1 | 29 June 2022 |
| | | | | MX | 2016009421 | A | 16 September 2016 |
| | | | | PL | 3096765 | T3 | 31 May 2019 |
| | | | | US | 10603300 | B2 | 31 March 2020 |
| | | | | US | 11433045 | B2 | 06 September 2022 |
| | | | | US | 2016-0361289 | A1 | 15 December 2016 |
| | | | | US | 2020-0179328 | A1 | 11 June 2020 |
| | | | | US | 2022-0378737 | A1 | 01 December 2022 |
| | | | | WO | 2015-110402 | A1 | 30 July 2015 |
| KR | 10-2014-0022086 | A | 21 February 2014 | CN | 103596564 | A | 19 February 2014 |
| | | | | CN | 103596564 | B | 04 May 2016 |
| | | | | EP | 2714032 | A2 | 09 April 2014 |
| | | | | EP | 2714032 | B1 | 16 January 2019 |
| | | | | ES | 2719656 | T3 | 11 July 2019 |
| | | | | JP | 2014-515396 | A | 30 June 2014 |
| | | | | JP | 5876570 | B2 | 02 March 2016 |
| | | | | KR | 10-1576589 | B1 | 10 December 2015 |
| | | | | TR | 201903445 | T4 | 21 March 2019 |
| | | | | US | 2014-0088079 | A1 | 27 March 2014 |
| | | | | US | 2015-0152075 | A1 | 04 June 2015 |
| | | | | US | 9034921 | B2 | 19 May 2015 |
| | | | | US | 9371303 | B2 | 21 June 2016 |
| | | | | WO | 2012-165914 | A2 | 06 December 2012 |
| | | | | WO | 2012-165914 | A3 | 28 March 2013 |
| KR | 10-2018-0039727 | A | 18 April 2018 | AR | 105844 | A1 | 15 November 2017 |
| | | | | AU | 2016-310535 | A1 | 22 February 2018 |
| | | | | AU | 2016-310535 | B2 | 19 August 2021 |
| | | | | BR | 112018003749 | A2 | 25 September 2018 |
| | | | | CA | 2996458 | A1 | 02 March 2017 |

Form PCT/ISA/210 (patent family annex) (July 2022)

19

**INTERNATIONAL SEARCH REPORT**
Information on patent family members

International application No.

**PCT/KR2023/001008**

| Patent document cited in search report | | | Publication date (day/month/year) | Patent family member(s) | | | Publication date (day/month/year) |
|---|---|---|---|---|---|---|---|
| | | | | CN | 107995862 | A | 04 May 2018 |
| | | | | CN | 107995862 | B | 22 October 2021 |
| | | | | CN | 107995862 | B8 | 03 December 2021 |
| | | | | EA | 201890592 | A1 | 28 September 2018 |
| | | | | EP | 3341024 | A1 | 04 July 2018 |
| | | | | JP | 2018-525424 | A | 06 September 2018 |
| | | | | JP | 2020-111598 | A | 27 July 2020 |
| | | | | JP | 6682621 | B2 | 15 April 2020 |
| | | | | JP | 6936893 | B2 | 22 September 2021 |
| | | | | MX | 2018002383 | A | 11 April 2018 |
| | | | | TW | 201717966 | A | 01 June 2017 |
| | | | | US | 10220017 | B2 | 05 March 2019 |
| | | | | US | 10709683 | B2 | 14 July 2020 |
| | | | | US | 2017-0056366 | A1 | 02 March 2017 |
| | | | | US | 2019-0142787 | A1 | 16 May 2019 |
| | | | | WO | 2017-032799 | A1 | 02 March 2017 |
| KR | 10-2017-0028452 | A | 13 March 2017 | AP | 2011005795 | A0 | 31 August 2011 |
| | | | | AR | 075424 | A1 | 30 March 2011 |
| | | | | AU | 2010-212865 | A1 | 21 July 2011 |
| | | | | AU | 2010-212865 | A8 | 17 November 2011 |
| | | | | AU | 2014-200258 | A1 | 30 January 2014 |
| | | | | AU | 2014-200258 | B2 | 12 May 2016 |
| | | | | AU | 2016-213789 | A1 | 01 September 2016 |
| | | | | AU | 2016-213789 | B2 | 18 January 2018 |
| | | | | AU | 2018-202278 | A1 | 26 April 2018 |
| | | | | AU | 2018-202278 | B2 | 02 January 2020 |
| | | | | BR | PI1013640 | A2 | 24 September 2019 |
| | | | | CA | 2751833 | A1 | 19 August 2010 |
| | | | | CA | 2751833 | C | 06 November 2018 |
| | | | | CL | 2011001757 | A1 | 28 October 2011 |
| | | | | CN | 102316875 | A | 11 January 2012 |
| | | | | CN | 104138370 | A | 12 November 2014 |
| | | | | CO | 6410306 | A2 | 30 March 2012 |
| | | | | EA | 021796 | B1 | 30 September 2015 |
| | | | | EA | 201101189 | A1 | 30 March 2012 |
| | | | | EC | SP11011275 | A | 30 September 2011 |
| | | | | EP | 2396005 | A1 | 21 December 2011 |
| | | | | GE | P20146120 | B | 25 July 2014 |
| | | | | HK | 1203814 | A1 | 06 November 2015 |
| | | | | IL | 213723 | A | 31 July 2011 |
| | | | | JP | 2012-517975 | A | 09 August 2012 |
| | | | | JP | 2015-042691 | A | 05 March 2015 |
| | | | | JP | 2017-052787 | A | 16 March 2017 |
| | | | | JP | 5896276 | B2 | 30 March 2016 |
| | | | | KR | 10-2011-0118668 | A | 31 October 2011 |
| | | | | MA | 33043 | B1 | 01 February 2012 |
| | | | | MX | 2011008214 | A | 29 November 2011 |
| | | | | MX | 344264 | B | 09 December 2016 |
| | | | | MY | 161472 | A | 14 April 2017 |
| | | | | NZ | 594485 | A | 26 July 2013 |

Form PCT/ISA/210 (patent family annex) (July 2022)

INTERNATIONAL SEARCH REPORT
Information on patent family members

International application No.

**PCT/KR2023/001008**

| Patent document cited in search report | Publication date (day/month/year) | Patent family member(s) | | Publication date (day/month/year) |
|---|---|---|---|---|
| | | PE | 20120002    A1 | 12 February 2012 |
| | | PH | 12016501607  A1 | 18 October 2017 |
| | | SG | 10201600204  A | 26 February 2016 |
| | | SG | 173587      A1 | 29 September 2011 |
| | | TN | 2011000413  A1 | 27 March 2013 |
| | | TW | 201040194   A | 16 November 2010 |
| | | TW | I478935     B | 01 April 2015 |
| | | UA | 106365      C2 | 26 August 2014 |
| | | US | 2011-0046087 A1 | 24 February 2011 |
| | | US | 2012-0196812 A1 | 02 August 2012 |
| | | US | 2014-0031301 A1 | 30 January 2014 |
| | | US | 2016-0158264 A1 | 09 June 2016 |
| | | US | 2017-0020907 A1 | 26 January 2017 |
| | | US | 2020-0069713 A1 | 05 March 2020 |
| | | US | 2021-0283156 A1 | 16 September 2021 |
| | | US | 2022-0313716 A1 | 06 October 2022 |
| | | WO | 2010-092123  A1 | 19 August 2010 |
| | | WO | 2010-092123  A8 | 30 June 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *J Nutr.*, July 2006, vol. 136 (7), 1940S-1946S
- *J Am Vet Med Assoc.*, 01 May 2002, vol. 220 (9), 1315-20

- **BROOKS et al.** *Journal of the American Animal Hospital Association*, 2014, vol. 50, 1-11